# EUROPEAN PATENT APPLICATION

(11) **EP 1 878 435 A1**
(43) Date of publication of application: **16.01.2008**
(21) Application number: 06745615.2
(22) Date of filing: 24.04.2006
(51) Int. Cl.: A61K 36/61

(54) **COMPOSITION FOR INDUCING SLEEP CONTAINING VEGETABLE ESSENTIAL OIL COMPONENT AS THE ACTIVE COMPONENT, TRANSDERMAL ABSORPTION TYPE SLEEP INDUCING AGENT CONTAINING THE COMPOSITION AND METHOD OF PRODUCING THE SAME**

(30) Priority: 26.04.2005 JP 2005128511
(71) Applicant: Nature Technology, Inc., Iwamizawa-shi Hokkaido 068-0853, (JP); Kamei, Junzou, Kanazawa-ku Yokohama-shi Kanagawa 2360012 (JP)
(72) Inventor: Karita, Takeshi, Ebetsu-shi Hokkaido; 0690824 (JP)
(74) Representative: Vossius & Partner
(86) International application number: PCT/JP2006/308537
(87) International publication number: WO 2006/118074

(57) **Abstract**

A water-absorptive resin is coated by geranyl acetate type Eucalyptus oil to form an essential oil coated resin, subsequently a carbon-coated particle is prepared by coating the essential oil coated resin with a carbon fine powder, which is an essential oil adsorption and desorption regulator; and then mixed with an adsorption promoter and a heat transfer inhibitor to form a sleep inducing composition. After that, the percutaneous sleep inducer comprising thereof is prepared.

## Description

### FIELD OF THE INVENTION

The present invention relates to a sleep inducing composition comprising an essential oil as an active ingredient, a percutaneous sleep inducer comprising thereof, and a method for producing thereof. In detail, the present invention relates to a percutaneous type sleep inducing composition having the essential oil as described above, the percutaneous type sleep inducer comprising the composition, and the methods for producing them.

### BACKGROUND ART

Essential oils generally have fragrances comprising compounds such as a variety of terpene, alcohols, and the like. It is known that some of the fragrant components in the essential oil, which is composed of these compounds, have effects such as an effect of relaxing tension and a calming effect; and therefore they have been used for a variety of ceremonies, and medical treatments or therapies.

There are many kinds of plants from which the essential oils are obtained, and among them, geranyl acetate type Eucalyptus is used as a raw material for obtaining the essential oil, because of its excellent fragrance. Alternatively, in Europe, wherein the essential oil has been used for the medical treatments or therapies from ancient age, and aromatherapy, in which the essential oil is taken per os or coated on skin, has been established as one of these therapies after 1930^{th}. The essential oil obtained from the geranyl acetate type Eucalyptus, hereinafter it is referred to as the geranyl acetate type Eucalyptus oil, is one of the essential oil frequently used in the aromatherapy.

On the other hand, since numbers of patients having disorder of sleep caused by stresses in modern days are increasing, hypnotics, sleeping agents, or sleep inducers are sometimes prescribed to improve symptoms.

Among these hypnotics, benzodiazepines are well known and the representative of them: There are mentioned, for example, agents having short time effect such as Triazolam, Temazepam, and Lormetazepam, and these having long time effect such as Flunitrazepam and Flurazepam. As the hypnotic other than Benzodiazepines, there are mentioned, for example, barbiturate, Chloral hydrate or Chlormethiazole. Alternatively, as a sleep inducer, there is mentioned, for example, Halcyon and the like.

Benzodiazepines improve affinity between γ-amino butyric acid (GABA) receptor in the central nervous system and GABA to increase receptor binding ratios, and decrease excitability of nerve cells. On the other hand, Benzodiazepines are active when they are administered per os, and most parts of them are decomposed by liver enzyme system to metabolites without inducing liver enzyme system. Furthermore, they are central nervous system depressants, but they are different from hypnotics other than Benzodiazepines, they are not lethal at the maximum dose, and the margin of their therapeutic range is not less than 100. This margin is more than 10 times higher compared with other sedatives such as barbiturate and so forth. This means that danger of respiratory suppression caused by intoxication is low. As a result, they are used as safety agents (herein below, it is referred to as the prior art 1, see non-patent reference 1).

Alternatively, the sleep inducer is conventionally developed and used as oral dosage from, because its active ingredient is not acceptable for percutaneous absorption. In this case, affections from the sleep inducer remains until the entire of the administered agent is eliminated as the metabolites or non-converted form of the agent from a patient body, even if the administration of the agent is discontinued. That is, the administration of the agent can not be immediately terminated.

As a percutaneous dosage form of which administration can be immediately discontinued and the amount of administration is less than that of the oral dosage form, there is a capsule type agent, wherein the essential oil is trapped in a carbon capsule (herein below, it is referred to as the prior art 2, see Patent document 1).

[Non-patent document 1] Atlas of Pharmacology, p.208, Bunkodo Publishing Co.
[Patent document 1] JP H08-109137 A

### DISCLOSURE OF THE INVENTION

### <PROBLEM TO BE SOLVED BY THE INVENTION>

Benzodiazepines shown in the prior art 1 is safer than other medicaments. However, they have side effects that responses to irritants becomes slow in a patient who is administrated them, thereby preventing rapid and proper responses.

Alternatively, when compatible benzodiazepines are administered to the patient in short time, sometimes the personality of the patient changes caused by appearing apathy and so forth. In contrast, when the drug is dosed by the patient in long term, sometimes addiction appears. Accordingly, there are strong needs for developing the safer medicament for the therapy.

The invention shown in the prior art 2 is excellent in the view point that the administration of the agent can be immediately discontinued, decreasing the dosage amount. However, in order to achieve antibacterial and anti-inflammation effects, the essential oils, 1-menthol, thymol, hinokithiol, citral and lavender oil are chosen and used in the prior art 2 so that these essential oils do not show any sleep inducing effects.

### <MEANS FOR SOLVING THE PROBLEM>

Under these situations, the inventors of the present invention found that the geranyl acetate type Eucalyptus oil show the sleep inducing effect when it is used in the percutaneous type pharmaceutical preparation, even through its concentration is very low; and then they completed the present invention.

That is, the present invention is a sleep inducing composition comprising a geranyl acetate type Eucalyptus oil as an active ingredient. The composition for the percutaneous agent comprises the essential oil obtained from plants as the active ingredient so that it neither causes any side effects nor addictions which are now at stake.

From the geranyl acetate type Eucalyptus described in below, an essential oil having excellent fragrance may be obtained by using steam distillation. Herein, the composition preferably comprises the carbon coated particle; which is produced that a water soluble resin is coated by the geranyl acetate type Eucalyptus oil obtained as described above to prepare the essential oil-coated resin, subsequently the resin is coated by a carbon fine powder, which functions as an essential oil desorbing regulator.

Furthermore, the present invention is a percutaneous type sleep inducer that comprises the geranyl acetate type Eucalyptus oil, an essential oil adsorbent, a free-water remover, an essential oil adsorbing and desorbing regulator, an exothermic agent, a heat transfer inhibitor, an absorption promoter, a base material for sheet forming, and a sheet for contact bonding. By applying the construction as mentioned above to the percutaneous agent, the geranyl acetate type Eucalyptus oil, which comprises the active ingredients, may be released in a manner called sustained release.

Additionally, the composition for the percutaneous sleep inducer wherein the active ingredient is uniformly distributed may be conveniently obtained as a sheet type agent; and then the composition is cut in a desired size. It means that the composition containing the amount of the active ingredients that matches to the physical condition of the patient may be produced.

Herein, the essential oil adsorbent is preferably poly-vinyl alcohol type water absorption resin of which saponification value is from 98.0 to 98.5. The free-water remover is preferably the acrylic type water-absorptive resin, which is capable of absorbing 400 to 800 times its volume of water based on the volume of the water-absorptive acrylic resin.

Additionally, as the essential oil-adsorbing and desorbing regulator, porous carbon material having surface area from 200 to 800 m² is preferably used. As the exothermic agent, a synthesized zeolite having a pore size from 0.1 to 0.8 nm is preferably used. Alternatively, as the heat transfer inhibitor, a polysaccharide compound is preferably used.

Furthermore, as the absorption promoter, mono terpene compound is preferably used, and the monoterpene is preferably 1-menthol or limonene. Additionally, as the base material for sheet forming, a thermoplastic resin having the saponification value, about 88.0.

The present invention is also a method for producing a sleep inducing composition comprising steps of; weighing predetermined weight of geranyl acetate type Eucalyptus oil; mixing the oil with an essential oil adsorbent to allow a coating of the essential oil on the essential oil adsorbent; adding a porous carbon material, which adsorbs and desorbs an essential oil, to an essential oil-coated adsorbent to allow a carbon coated particle being coated by the porous carbon material; mixing homogeneously a predetermined amount of the carbon coated particle, an exothermic agent, a heat transfer inhibitor, and a base material for sheet forming, to prepare a layer having even thickness, and then heated them to form a composition for sleep inducing agent; cutting said composition for sleep inducing agent, which is percutaneous agent, in a predetermined size of a piece to sandwich it by using two sheet type materials; and thermal bonding of four sides of the sheet type materials.

By using the producing method for the present invention as described above, it may be obtained a sheet type of the composition for the percutaneous sleep inducer comprising the desirable amount of the active ingredient.

Furthermore, by using the method, the geranyl acetate type Eucalyptus oil adsorbed on the surface of the essential oil adsorbent is coated by the porous carbon material; and then these carbon coated particle are covered by using two sheet type materials. By employing such constitution, the percutaneous agent may be produced; wherein the geranyl acetate type Eucalyptus oil comprising the active ingredients does not directly contact with the skin of the patient, continuing the release of the geranyl acetate type Eucalyptus oil in long time. The percutaneous sleep inducer has an advantage that it does not appear any side effects held by the prior sleep inducer or hypnotics; and another one that it is easily removed from the applied location to discontinue the administration.

### <EFFECT OF THE INVENTION>

As explained above, the percutaneous sleep inducer of the present invention is highly safe, and may be used with no particular side effects. Furthermore, it has the advantageous effect that the location on which the sleep inducer is not particularly limited.

Furthermore, according to the method for producing the percutaneous sleep inducer, the percutaneous type sleep inducer may be conveniently produced. Particularly, it has the advantageous effect that it gives high sustained release, because it employs the essential oil desorbent in the carbon coated particle.

According to the method for producing the percutaneous sleep inducer of the present invention, the dose of the pharmaceutical agent may be adjusted to each patient to be administered the agent by cutting the composition for the sleep inducer into the proper size.

Furthermore, according to the method for producing the present invention, the plaster containing the desired amounts of the active ingredients also may be produced by changing the amounts of the carbon coated particle.

### BRIEF DESCRIPTION OF THE DRAWINGS

Fig. 1 shows the prolongation of sleep, when percutaneous agents containing each essential oil are used;
Fig. 2 shows the relationship between the prolongation of sleep and the contents of geranyl acetate type Eucalyptus oil in the agent, when the percutaneous agent is used;
Fig. 3A shows a time dependent change, when the percutaneous agent including the geranyl acetate type Eucalyptus oil is applied on a normal skin;
Fig. 3B shows the time dependent change, when the percutaneous agent including the geranyl acetate type Eucalyptus oil is applied on a damaged skin;
Fig. 4A shows weight change of male rats during a percutaneous toxicity test;
Fig. 4B shows weight change of female rats during the percutaneous toxicity test;
Fig. 5A shows change of feeding amount by the male rats during the percutaneous toxicity test; and
Fig. 5B shows change of feeding amount by the female rats during the percutaneous toxicity test.

### BEST MODE FOR CARRYING OUT THE INVENTION

The present invention will now be explained in detail herein below.
Main habitat of the geranyl acetate type Eucalyptus oil used in the present invention is south area of New south Wales in Australia, and it may be obtained from small leaves and twigs of Eucalyptus macarthuri H. Deane et J. H. Maiden by steam distillation at the rate of yield is 0.15 to 0.5 %.

The geranyl acetate type Eucalyptus oil contains the following components: geranyl acetate (about 70%), geraniol (about 3%), eudesmol (about 16%), and other aliphatic aldehydes such as isovaleraldehyde, p-cymene, camphene, dipentene and the like.

The geranyl acetate type Eucalyptus oil may be used commercially available one, for example, produced by Ogawa & Co., Ltd.

In order to compare with the sleep inducer of the present invention, percutaneous agents including one essential oil are produced; wherein the essential oil is selected from the group consisting of Hop oil, Sandal wood oil, cineol type Eucalyptus oil, and Rosemary oil.

Hop oil is obtained from Hop (Humulus lupulus L.) belongs to Moraceae, and is main habitat is areas from Europe to west Asia. In Hop, there are following two species used for medicaments: one is Kanamugura (Humulus japonicas Sieb. et Zucc.) which naturally grows in Japan, and another one is Humulus americanus Nutt. which naturally grows in the United States and used similarly.

Hop is a dried staminate female inflorescence that is harvested before fertilization. Hop concrete is obtained from Hop by solvent extraction using organic solvents such as ether and benzene as a solid wax. Hop concrete is further extracted by using alcohol, and then remains are obtained after vacuum distillation to recover the alcohol. The remains are referred to as Hop absolute.

Alternatively, Hop oil is obtained from Hop by steam distillation. Hop oil contains soft resin as active ingredients; wherein the soft resin generates α acid (humulons), or β acid (lupulones). As fragrant ingredients, the following compounds are included: myrcene (50 %), humulene (15 to 25 %), caryophyllene, farnesene, linalol, geraniol, myrcenol and its esters, methylnonylketone, lupalol, luparenol, and the like.

Sandal wood oil is an essential oil obtained from roots and lumber of Santalum album L. by steam distillation and Santalum album L. belongs to Santalaceae, of which habitat is east India, south India, Malay, Celebes, and so forth. As components, is contains α-santalol, β-santalol (peculiar component, 90 %), santalene, santenone, santenol, teresantalol, santalone, and so forth.

Cineole type Eucalyptus oil may be obtained from leaves of Eucalyptus globulus Labill., of which habitat is north America, Mexico, Africa and south Spain but its origin is Tasmania, by steam distillation. As the components, is contains cineol (70 to 80 %), α-pinene, camphene, pinocarveol, pinocarvone, berbenone, carvone, eudesmol, and aliphatic aldehyde having C₄~C₆.

Rosemary oil may be obtained from flower, leaves or entire of the plant, of which habitat is Spain, Yugoslavia, France or Italy, by steam distillation. As the components, it contains borneol, bornyl acetate, campher, cineol, and other terpenes.

The essential oils described above such as Hop oil, Sandal wood oil, cineol type Eucalyptus oil, and Rosemary oil, may be used commercially available ones from manufacturers, such as Ogawa & Co. Ltd., and the like.

*l*-Menthol (5-methyl-2-(1-methylethyl)cyclohexanol) is generally called menthol. *l*-Menthol has twelve chemical isomers, but synthetic or natural *l*-menthol has solely cool flavor that is peculiar to menthol. *l*-Menthol is colorless pillar or needle, and soluble in ethanol but insoluble in water. It gradually sublimate in room temperature.

In order to obtain natural *l*-menthol, menthol oil is cooled and centrifuged to separate deposited crystalline, and then menthol is obtained. Synthetic one is obtained from d-citronellal, which is obtained from citronella oil by fractional distillation, changed to *l*-isopulegol, and then hydrogenated. Alternatively, myrcene obtained from pinene is used as the raw material to synthesize an optical active citronellal by using a special catalyst. By this method, menthol is asymmetrically synthesized without optical resolution. Menthol is also obtained from a mixture of menthol, which is obtained from thymol with hydrogenation, by optical resolution.

The essential oil adsorbent used in the percutaneous sleep inducer of the present invention is defined as an adsorbing carrier that adsorbs geranyl acetate type Eucalyptus oil as described above. As the essential oil adsorbent, poly vinyl alcohol (PVA) type water soluble resin having the saponification value from 98.0 to 98.5 is preferably used. If the saponification value is less than 98.0, the surface of the carrier resin is gelatinized and it loses functions as the adsorbent carrier. However, if the saponification value is in the range between 98.0 and 98.5, the surface of the carrier resin is not gelatinized so that it may keep the function as the stable carrier adsorbent.

Particularly, there is mentioned the resin, for example, Shin-Etsu Poval C-17GP and Poval (A) (both of them are provided by Shin-Estu Chemical Co. Ltd.), and so forth. Among them, Shin-Etsu Poval C-17GP or Poval A is preferably used.

A free-water remover used in the percutaneous sleep inducer of the present invention is defined as the material removing water on the skin surface on which the percutaneous agent is placed. An acrylic type water-absorptive resin is preferably used, because such resin has high performance of water absorption in general, and also has good adhesive properties when it is subjected to a heating process for producing the composition described in below.

The acrylic type water-absorptive resin may absorb water generated on the particular space of the skin surface during the percutaneous agent is applied on, and it is capable of absorbing 400 to 800 times of its volume of water based on the volume of the water-absorptive resin. If the capacity of the water-absorption is lower that 400 times, the resin is not capable of absorbing entire of the generated water, but the capacity over 800 times is not necessary. For example, there are mentioned Sanfresh (Sanyo Chemical Industries Ltd.), Aquakeep (Registered trademark, Sumitomo Seika Chemicals Co., Ltd.), and the like. Among them, Sanfresh having fractured form is preferably used because it has good adhesive property compared with Aquakeep having particle form.

An essential oil adsorbing and desorbing regulator used in the percutaneous sleep inducer of the present invention is defined as a porous carbon material that coats the surface of the layer formed by the above-mentioned essential oil on the essential oil adsorbent for regulating adsorption and desorption of the essential oil. Specifically, there are mentioned, for example, activated charcoal that adsorbs a variety of molecules. The activated charcoal having 200 to 1,000 m²/g of surface area is preferably used; because the amount of the essential oil adsorbed to the charcoal per weight is a little and thereby desorbing the essential oil becomes easy. The activated charcoal having 400 to 800 m²/g of surface area may be more preferably used.

In the present invention, as the above-mentioned activated charcoal, which is finely divided particle, commercially available one may be used. There are mentioned, for example, Shirasagi P (Takeda Pharmaceutical Company Ltd.) and so forth. Among them, Shirasagi P is preferably used, because the adsorption area is not too large and the cost is reasonable.

An exothermic agent used in the percutaneous sleep inducer of the present invention is defined as a material that adsorbs moisture in the air and then generates adsorption heat. By using the thermal energy generated when the moisture is adsorbed, the essential oil adsorbed onto the carrier adsorbent is desorbed. Specifically, there is mentioned zeolite as an example.

The zeolite used in the percutaneous sleep inducer of the present invention is synthetic one that does not contain any metal and it has pore size from 0.1 to 0.8 nm, because the energy for adsorption and desorption is properly supplied. The zeolite has pore size from 0.3 to 0.4 nm is more preferably used. Commercially available zeolite may be used when it has such pore sizes, and specifically, Zeolum (Tosoh Corporation) and so forth are mentioned as examples.

A heat transfer inhibitor used in the percutaneous sleep inducer of the present invention is defined as a compound for inhibiting to transfer the heat, which is caused by the free-water adsorption onto the free-water adsorbent. Specifically, there are mentioned, for example, polysaccharide compound such as chitosan and cellulose and so forth.

When a dye is contained in the pharmaceutical preparation, use of chitosan gives an advantage that chitosan may be used as the carrier of such a dye. In order to inhibit the heat transfer, cellulose and the like are used instead of chitosan.

An absorption promoter used in the percutaneous sleep inducer of the present invention is defined as monoterpene that functions to improve to absorb the essential oil in the inducer. As the absorption promoter, *l*-menthol and other terpene compound are specifically mentioned as the example, and commercially available one may be used. Among them, *l*-menthol has the advantageous effect that it removes the free-water remained on the skin, and then arranges circumstances for the essential oil to be absorbed through the skin.

A base material for sheet forming used in the percutaneous sleep inducer of the present invention is defined as the base material to form a sheet type sleep inducing composition. As the base material, the thermoplastic resin having the saponification value, about 88.0, is preferably used. In order to form the percutaneous sleep inducer of the present invention, the sleep inducing composition should be subjected to a heating process. In this time, the resin should have preferable properties that neither release the geranyl acetate type Eucalyptus oil from the carbon coated particle nor stuck spaces among the resin particle, even though the adhesion is performed under lower temperature, such as about 180°C.

Specifically, for example, Gohselan L-0301 (Registered trademark, Nippon Synthetic Chemical Industry Co., Ltd.) is preferably used, because of the good adhesive properties at low temperature about 180°C.

The percutaneous sleep inducer of the present invention may be plaster type preparation through the following process: the essential oil coated particles manufactured as described above are mixed, and then prepare plasters by using an oleaginous base used for the base of suppository and ointment, or water-soluble ointment base is used as the base.

As the oleaginous base, there are mentioned, for example, lard, beef tallow, fatty oil, hydrocarbons, higher alcohols, higher fatty acid, higher fatty acid esters, glycols, plant oils, animal oils and so forth.

Among them, as the hydrocarbon, there are mentioned, for example, liquid paraffin that is a mixture of a variety of the hydrocarbons, paraffin having branched chain (Commercial name, Isopar, registered trademark, Exxon Mobil Corporation), solid paraffin, white soft paraffin, and so forth.

As higher fatty acid, there are mentioned, for example, hexanoic acid, enanthic acid, caprylic acid, pelargonic acid, undecylic acid, lauric acid, tridecylic acid, myristic acid, pentadecylic acid, palmitic acid, heptadecylic acid, stearic acid, oleic acid, and so forth.

As the higher fatty acid ester, there are mentioned, for example, fatty acid ester such as myristyl myristate, myristyl palmitate, stearyl stearate, ceryl lignocerate: lanolin; lately describing waxes; glyceryl laurate, glyceryl monomyristate, glyceryl monooleate, glyceryl monostearate, glyceryl dilaurate, glyceryl dimyristate, glyceryl distearate, glyceryl trilaurate, glyceryl trimyristate, glyceryl tristearate, and so forth.

As fatty oil, there are mentioned, for example, the plant oil such as soybean oil, camellia oil, rape seed oil, peanut oil, sesame oil, and safflower oil; animal oil such as mink oil, egg yolk oil, squarane, fish oil, whale oil, and liver oil; and hardened oil produced by hydrogenation thereof.

As the wax, there are mentioned, for example, carnauba wax, yellow bee wax, white beeswax, and so forth. As the petrolatum, there are mentioned, for example, yellow petrolatum, white soft paraffin and so forth.
White soft paraffin is preferably used, because it is compatible to almost pharmaceutical drug with no change. According to the description on the Japan Pharmacopoeia, white soft paraffin is combined with white bee wax and sorbitan sesquioleate to prepare white ointment and then absorption promoter is properly added to prepare the plaster.

Alternatively, as the water soluble base, there are mentioned, for example, carboxyvinylpolymer, hydroxypropylcellulose (herein below, it is also referred to as simply "HPC"), macrogol, methylcellulose, and so forth. A commercially available water-soluble base, Hiviswako (Registered trademark, Wako Pure Chemical Industries, Ltd.) is preferably used. When Polyethylene glycol 400 and Polyethylene glycol 4000 are mixed at equal ratio, the macrogol ointment is prepared and may be used as the water-soluble base. Alternatively, HPC having good compatibility with propylene glycol may be used as the base.

Note that skin irritation is reduced by adding, at least, diisopropyl-ethanolamine and diisopropyladipate as neutralization agents, when an acidic water-soluble polymer is used as the base.

For example, *l*-menthol or limonene may be preferably used, because the main ingredient of the present invention, aliphatic essential oils obtained from plants, are combined with oleaginous base, if necessary. When the main ingredient is combined with the water-soluble base, the absorption promoter is not necessary. However, *l*-menthol may be used in the amount as mentioned above.

The percutaneous agent of the present invention is prepared by using the essential oil, the essential oil adsorbent, the free-water remover, the essential oil adsorbing and desorbing regulator, the exothermic agent, the heat transfer inhibitor, the absorption promoter, and the base material for sheet forming, as described above. The method for producing thereof is explained in below.

At first, a desired amount of the essential oil is weighed, and then it is mixed with PVA to coat the surface of PVA particle in a proper size vessel. Subsequently, the desired amount of the activated charcoal is weighed and added to cover the surface of the essential oil-coated PVA to prepare the charcoal coated particle A.

Next, menthol is weighed. Since menthol is solid, the weighed menthol is referred to as the particle B.

These two particles A and B are mixed, and the heat transfer inhibitor and the base material for sheet forming are sequentially mixed to prepare homogenous mixture. After that, the mixture is sandwiched by two sheets for contact bonding, and heated to prepare the sheet type of the composition for the percutaneous sleep inducer of the present invention.

After the mixture is sandwiched by the sheets for contact bonding, the sandwiched mixture is preferably heated from about 160 to about 200 °C, more preferably about 180 °C. By heating the sandwiched mixture at the temperature of the above-mentioned range, the base material for sheet forming, the resin, is adhered each other, keeping spaces among them. Since the spaces works as isles for the essential oils released from the charcoal coated particle to the skin surface, on which the percutaneous sleep inducer of the present invention is applied, the essential oil is delivered to the skin and absorbed percutaneously. On the other hand, when the mixture is heated higher temperature, 230 °C, the spaces among the resin are filled because the resins are melted. Therefore, the essential oils described above are incapable of delivering to the skin.

As the sheet for contact bonding, Kasenshi paper (basis weight 18 to 20 g) is preferably used, and the use of Kasenshi paper has the advantage that the ratio of contact bonding of carbon coated particles is high.

The sheets contact bonded to the composition is sandwiched with two sheet type materials made of nonwoven fabrics with proper sizes; and then four sides of the sheets are heat-sealed to prepare a piece of the percutaneous sleep inducer of the present invention.

Alternatively, the sheet type material is preferably selected from the group consisting of paper, woven fabric and nonwoven fabric. Among them, the nonwoven fabric is more preferable because of its good permeability. The essential oil contained in the percutaneous sleep inducer is released as gaseous state, and it flows in the spaces among the particles of the base material for sheet forming to go out by permeating the fabric.

Plasters are prepared in below. At first, the desirable amount of the essential oil is weighed and mixed with PVA to coat the surface of PVA. After that, PVA with the essential oil coat is mixed with the desirable amount of the activated charcoal as described above to form the carbon-coated particle A. Subsequently, the desirable amount of menthol is weighed, and it is refereed to as the particle B.

These particles A and B are mixed, and then the mixture is mixed with the oleaginous base or the water-soluble base, and spread on a sheet made of the paper, the plastic film, and so forth.

As explained above, the composition for the sleep inducer and the sleep inducer of the present invention are prepared.

### EXAMPLES

The present invention will now be described by the following examples in detail. However, the present invention is not limited to the examples.

### (Example 1)

### (1) Reagents

In order to prepare the composition for the percutaneous sleep inducer, the percutaneous sleep inducer, a control agent, and placebo, the following reagents are used.

### (1-1) Essential oils

Geranyl acetate type Eucalyptus oil and *l*-menthol are purchased from Ogawa & Co., Ltd. Hop oil, Sandal wood oil, cineol type Eucalyptus oil and Rose oil are also purchased from Ogawa & Co., Ltd.

### (1-2) Others

As PVA, Shin-Etsu Poval C-17GP is purchased from Shin-Etsu Chemical Co., Ltd., and Gohselan L-3031 is purchased from Nippon Synthetic Chemical Industry Co., Ltd. As the acrylic type water-absorptive resin, Sanfresh (Registered trademark) is purchased from Sanyo Chemical Industries, Ltd. As the activated charcoal, Shirasagi P is purchased from Takeda Pharmaceutical Company Limited, and as the zeolite, Zeolum (registered trademark) is purchased from Tosoh Corporation. As the chitosan, Koyo Chitosan is purchased from Koyo Chemical Company Limited.

Kasenshi paper is purchased from Nippon Daishowa Paperboard Co., Ltd., and the nonwoven fabric is purchased from Nisshinbo Industries, Inc.

### (Example 2) Preparation of the composition for the percutaneous sleep inducer, and the percutaneous sleep inducer

### (2-1) Preparation of the carbon-coated particle

The carbon coated particle is prepared according to the recipe shown in the following table 1. Note that each piece of the agent is regulated so as to contain 1.1 mg of each carbon coated particle prepared by using the amount shown in the table 1, when the pharmaceutical agent (Name of the agent is SD-A, SD-B, SD-C, and SD-K) is prepared.

**(Table 1)**

| Contents(weight(mg)) | No. of the carbon coated particle | | | |
|---|---|---|---|---|
| | 1 | 2 | 3 | 4 |
| No. of Agent | SD-A | SD-B | SD-C | SD-K |
| Hop oil | 1,500 | - | - | - |
| Geranyl acetate type Eucalyptus oil | - | 1,500 | - | - |
| Sandal wood oil | - | - | 1,500 | - |
| Cineol type Eucalyptus oil | - | - | - | 1,500 |
| Lavender oil | - | - | - | - |
| PVA(Shin-EtsuPoval) | 100 | 100 | 100 | 100 |
| Activated Charcoal | 2,000 | 2,000 | 2,000 | 2,000 |
| Total | 3,600 | 3,600 | 3,600 | 3,600 |

For example as SD-B, geranyl acetate type Eucalyptus oil is weighed at the amount shown in the table 1 and put them in 500 ml of a transparent glass closed vessel. PVA (Shin-Etsu Poval C-17GP) is added into the vessel and mixed with the Eucalyptus oil at the room temperature to prepare the essential oil coated particle. Subsequently, the activated charcoal is added into the vessel in the amount shown in the table 1 and then mixed. The carbon coated particle prepared as mentioned above is then stored in a tight sealed vessel, for example, a glass vessel with a ground-glass stopper, at room temperature.

### (2-2) Preparation of the composition for the percutaneous sleep inducer

The carbon coated particles prepared in the above-mentioned (2-1) are mixed with the base material at the amount as shown in the following table 2, and the composition for the percutaneous sleep inducer is prepared.

**(Table 2)**

| | | combined amount (g) | | | |
|---|---|---|---|---|---|
| Name of Agent | | SD-A | SD-B | SD-C | SD-K |
| Essential oil coated particle | | 100 | 100 | 100 | 100 |
| Base | Sanfresh | 41 | 41 | 41 | 41 |
| | L-menthol | 19 | 19 | 19 | 19 |
| | Zeolum | 6 | 6 | 6 | 6 |
| Activated Charcoal | | 13 | 13 | 13 | 13 |
| Koyo Chitosan | | 11 | 11 | 11 | 11 |
| Gohselan L-0301 | | 120 | 120 | 120 | 120 |
| Total | | 310 | 310 | 310 | 310 |

The base and the above-mentioned carbon coated particles are mixed in below.
At first, Sanfresh (Sanyo Chemical Industries, Ltd.), Zeolum (zeolite, Tohsoh Corporation), *l*-menthol (Ogawa & Co., Ltd.), and Koyo Chitosan (Koyo Company Ltd.) are sequentially in this order added and mixed, and prepared the base.
Next, the carbon coated particles prepared as shown in the above-mentioned (2-1) are weighed in the amount shown in the table 2, and mixed with the base. After that, Koyo Chitosan, Shiarasagi P and Gohselan are added and mixed. Note that the ratio of the essential oil in the composition lost during the contact bonding under heating is assumed 15 % to calculate the weight.

Herein, the mixture containing the base and the carbon coated particles are spread on the sheet for contact bonding so as to have even thickness, and then another sheet for contact bonding is placed on them to sandwich the mixture. The sandwiched mixture is then subjected to contact bonding under heating, and formed as the sheet type composition for sleep inducer. The sheet type composition is, for example, cut in a proper size to form pieces and sandwiched with the nonwoven fabric that is also cut in the proper size to cover the piece. After that, four sides of the nonwoven fabric is heat sealed to prepare the percutaneous sleep inducer of the present invention. Each recipe is given names as SD-A, SD-B, SD-C, and SD-K in sequence, according to the sequence in number.

Placebo is prepared as the same as mentioned above, using black paper not including geranyl acetate type Eucalyptus oil instead of the sheet type composition of the present invention.

### (EXAMPLE 3) Evaluation of the sleep inducing effect by the percutaneous agent including each essential oil

Evaluation of the sleep inducing effect by the percutaneous preparations including each essential oil is performed the duration of pentobarbital-induced loss of righting reflex (LORR) as an index.

### (3-1) Animals and so forth

As the test animals, ICR mice, 5 weeks are purchased from Tokyo Laboratory animals Co. Ltd. After 1 week condition, they are divided into 10 mice per group. Pentobarbital is purchased from Dainippon Pharmaceutical Co., Ltd.

### (3-2) Test method and effects

Duration of loss of righting reflex (LORR) induced when pentobarbital is administered in the mouse is measured, and evaluated the duration as sleeping time.
On one day before the test, the mouse is anesthetized by using pentobarbital (70mg/kg, i. p.), and then shaved hair on its back to secure the place on which the agent to be tested as prepared in the example 2.
The test is carried out in a separated room of which temperature is kept at 24 °C from 9 to 15 o'clock.
On the test day, each preparation is put on the place of the mice for administration. Three hours later, they are also administered pentobarbital (50mg/kg, i.v.), and the mice that lost righting reflex put on the V-shape stand in supine position.
When the mice are wake up three times on the V-shape stand in 30 minutes, it is evaluated as the recover from LORR. Duration of LORR is from losing righting reflex after the mice are anesthetized to the recovery from LORR.

Preparations SD-A, SD-B, SD-C and SD-K, and placebo are attached on the back, on which the hair was shaved, of the different mice, and examined LORR function of them.

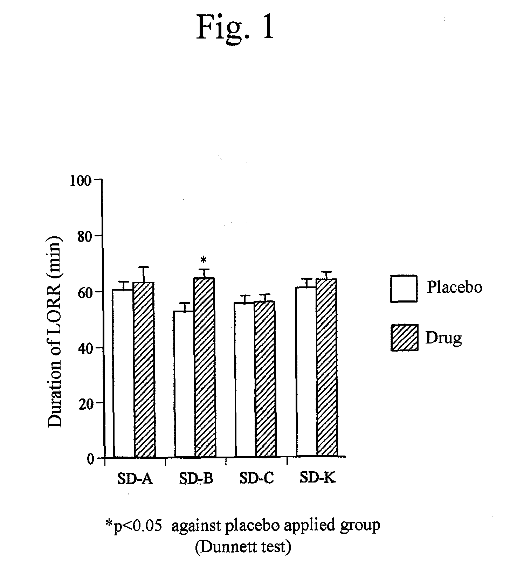

Results of attaching SD-A, SD-B, SD-C and SD-K, and the placebo are shown in Fig. 1. As shown in Fig. 1, SD-B (contains geranyl acetate type Eucalyptus oil) shows the significant sleeping time prolongation (in Fig. 1, * is given. p<0.05). According to the result, it is demonstrated that geranyl acetate type Eucalyptus oil has sleep inducing effect.

### (3-3) Relationship between dosage of SD-B and the sleep inducing effect

As shown in the above-mentioned (3-2), the significant sleep inducing effect is demonstrated when the geranyl acetate type Eucalyptus oil is used. Accordingly, dose dependency of the geranyl acetate type Eucalyptus oil and the sleep prolongation effect is studied by changing the dosage of SD-B as half, equal and double. The preparations of the dosage are referred to as SD-B1/2, SD-B1, and SD-B2, respectively.

Sizes of the sheets of SD-B1/2, SD-B1, and SD-B2 are 2cm x 2 cm. The amount in each piece is 0.55 mg in SD-B1/2, 1.1 mg in SD-B1, and 2.2 mg in SD-B2.
For the mouse, they are cut in half, and the amounts in the pieces are respectively 0.275mg, 0.55mg, and 1.1mg.

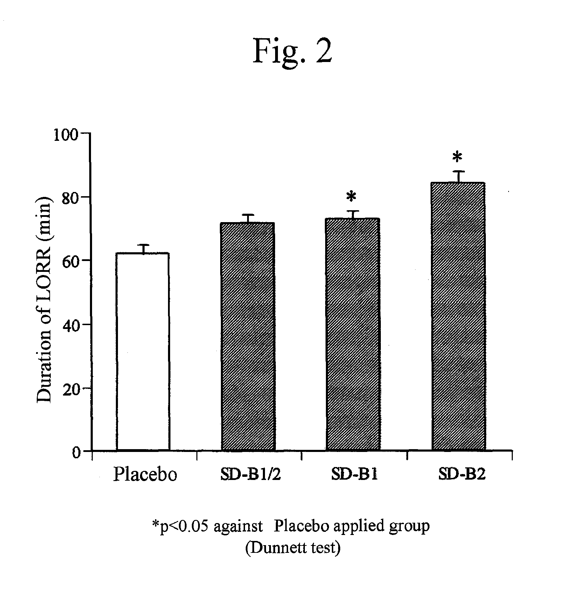

Similarly to (3-2), SD-B1/2, SD-B1, and SD-B2 are attached on the back of the mice, which are administered pentobarbital, and observed the prolongation of the duration of LORR. Results are shown in Fig. 2.

In the group of SD-B1/2, it is not demonstrated the significant prolongation of the duration of LORR to that of placebo (In fig. 2, * is given. p < 0.05). However, in the both groups of SD-B1 and SD-B2, it is demonstrated the dose-dependent significant prolongation of the duration of LORR.

### (EXAMPLE 4) Primary irritant test to skin of SD-B

As a GLP ministerial ordinance, the ministerial ordinance of which title is "the ministerial ordinance related to the standard for performing non-clinical test for safety of medications" (March 26, 1997, the 21 st ministerial ordinance by Ministry of Health, Labor and Welfare) is applied. As a guide line, Guidebook for Application for Manufacturing Approval of Cosmetics and Quasi drug (4th Edition) and Safety Guideline of Cosmetics 2001 are applied.

### (4-1)Test animals and so forth

### (1) Test animals

Male, Japanese white rabbit, conventional (2.16~2.36kg, 11 weeks) are purchased from Saitama Laboratory Animals Co. Ltd. These rabbits are conditioned in 7 days, and subjected to the following test.

### (2) Samples tested

As the sample preparation, SD-B describe above is used. As a control, Cont that is prepared similarly to the SD-B without the composition of the present invention.

### (3-2) Test method and results

### (1) Formation of the place subjected to the test

Hair on the both sides of the back of the rabbit is shaved by using the shaver (THRIVE ANIMAL CLIPPER Model 6000AD, Daito Electric Co., Ltd.) on the day before the test. One side of the shaved back is used as normal skin and the other side is used as damaged skin after abrading to form three projected parallels on keratin by using 18G needle not hurt corium.

### (2) Administration method of the test samples

SD-B and Cont prepared as mentioned above are patched on the places for the test respectively, and performed obstructive test for 24 hours by using non-systemic adhesive plaster (BLENDERM, Registered trademark, 3M Company).

In order to improve the adhesion properties, out side of BLENDERM is fixed by using tacky sponge plaster (trade name: Microform, 3M Company) and stretching plaster (trade name: Silkytex, Alcare Co., Ltd.).

### (3) Decision of the irritant strength and standard

Three time points, after 1 hour, 24 hour and 48 hour from the attachment of the samples and placebo, erythema, formation of crustal impetigo, and formation of edema are decided according to the Draize's Decision shown in the following Table 3.

**(Table 3)**

| Conformity | Erythema and formation of crustal impetigo | Formation of edema |
|---|---|---|
| 0 | No Erythema | No edema |
| 1 | Slight erythema (faintly recognized) | Slight edema (faintly recognized) Slight edema (faintly recognized) |
| 2 | Clear erythema | Light (clearly distinguished from periphery) |
| 3 | From medium to strong erythema | Medium edema (swelled about 1 mm) |
| 4 | Strong erythema with deep red and light formation of crustal impetigo (damage reaches deep part) | Strong edema (swelled more than 1 mm, and spread out in a periphery) |

Based on the standard, both time points, after 1 hour and 48 hour from the attachment of the samples and placebo, the degree of the erythema and the formation of crustal impetigo, as well as the formation of the edema are determined. Reaction strengths are scored and mean of the score are the primary irritant indexes (P. I. I.).
The primary irritant properties are decided based on the evaluation shown in Table 4.

**(Table 4)**

| Evaluation point | Evaluation class |
|---|---|
| 0 | No Stimulant |
| 0 < P.I.I. < 2 | Weak Stimulant |
| 2 < P.I.I. < 5 | Medium Stimulant |
| 5 < P.I.I. < 8 | Strong Stimulant |

### (4) Decision result

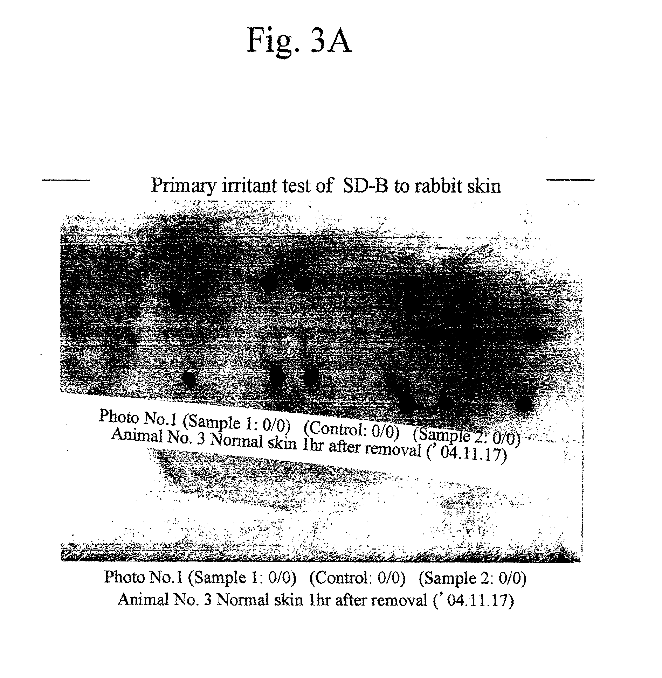

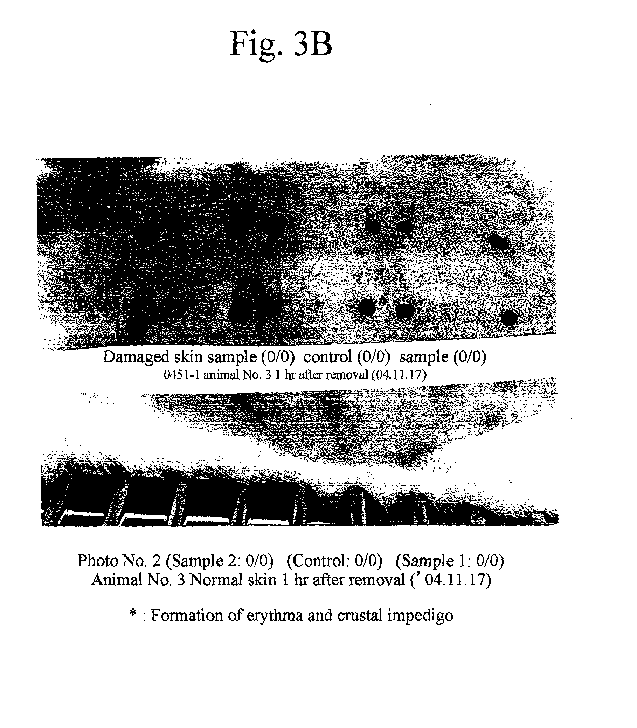

The score of the primary irritant index and their evaluations depending on individual animal score are respectively shown in Tables 5 and 6. Alternatively, photographs of the skin reaction 1 hour after removal of the test sample or placebo are shown in Fig. 3A and 3B.

**(Table 5)**

| Animal No. | Normal skin | | | | Damaged skin | | | |
|---|---|---|---|---|---|---|---|---|
| | Erythema and formation of crustal impetigo | | Formation of edema | | Erythema and formation of crustal impetigo | | Formation of edema | |
| | 1hr | 48hr | 1hr | 48hr | 1hr | 48hr | 1hr | 48hr |
| 1 | 0 | 0 | 0 | 0 | 0 | 0 | 0 | 0 |
| 2 | 0 | 0 | 0 | 0 | 0 | 0 | 0 | 0 |
| 3 | 0 | 0 | 0 | 0 | 0 | 0 | 0 | 0 |
| Mean | 0 | | 0 | | 0 | | 0 | |
| Strength | 0 | | | | 0 | | | |
| P.I.I. | 0 | | | | | | | |

**(Table 6)**

| Animal No | Items for Decision | Normal skin | | | Damaged skin | | |
|---|---|---|---|---|---|---|---|
| | | 1hr | 24hr | 48hr | 1hr | 24hr | 48hr |
| 1 | Erythema and formation of crustal impetigo | 0 | 0 | 0 | 0 | 0 | 0 |
| | Formation of edema | 0 | 0 | 0 | 0 | 0 | 0 |
| 2 | Erythema and formation of crustal impetigo | 0 | 0 | 0 | 0 | 0 | 0 |
| | Formation of edema | 0 | 0 | 0 | 0 | 0 | 0 |
| 3 | Erythema and formation of crustal impetigo | 0 | 0 | 0 | 0 | 0 | 0 |
| | Formation of edema | 0 | 0 | 0 | 0 | 0 | 0 |

As shown in Table 5, there are neither observed any erythema, the formation of crustal impetigo, and the formation of edema on the normal skin nor the damaged skin, when the tested preparations are used after 48 hours from the beginning of the test.

Alternatively, time dependent change after attachment is individually observed, but any changes are not observed in the time point at 1 hour, 24 hour, or 48 hour. Furthermore, the erythema and the formation of crustal impetigo, as well as the formation of edema are not observed.

### (EXAMPLE 5) Percutaneous toxicity test of SD-B in rat during 21 days repeat administration

The ministerial ordinance, "the ministerial ordinance related to the standard for performing non-clinical test for safety of medications" (March 26, 1997, the 21st ministerial ordinance by Ministry of Health, Labor and Welfare), Guideline for toxicity test necessary for Application (or import) of medications (September 11, 1989, the 24^{th} Notice by Drug Evaluation 1), Guidebook for Application for Manufacturing Approval of Cosmetics and Quasi drug (4th Edition) and Safety Guideline of Cosmetics 2001, and OECD Guidelines for the Testing of Chemicals (TG410, employed on May 12, 1981) are applied.

### (4-1) Test animals and so forth

### (1) Test animals

Sixteen male and female Slc: Wister SPF rats (8 weeks) are purchased from Japan SLC; Inc. After conditioning in 6 days, they are subjected to the test. Body weights of the male rats are from 170 to 188 g, and those of the female rats are 120 to 139 g, when they are sipped. Those of them at the beginning of the administration are 214 to 229 g of the male rats, and 145 to 163 g of the female ones, except control ones.
A couple of male or female rats are separately placed per cage, and water (tap water) and feed (pellet type MF, Oriental Yeast Co., Ltd.) are freely fed.

### (2) Test preparations

As the test preparation, SD-B as described above is used. As the control sample, Cont that is prepared similarly to SD-B except no use of the sheet type composition of the present invention is used.
The above-mentioned male rats are separated in control group (n = 5) and test group (n = 5), and also female rats are separated similarly. The percutaneous administration is performed for 21 days, according to the following method.

### (3) Test method

### (1) Formation of the site on which test samples and Cont are attached, and administration of them

Hair on the back of the rats is shaved by using the shaver (THRIVE ANIMAL CLIPPER Model 6000AD, Daito Electric Co., Ltd.) on the day before the test, and then the hair is shaved in every a couple of days.
Test sample is directly attached on the site, and performed obstructive attachment for 24 hours by using an adhesive plaster (Dermapore, Alcare Co., Ltd.). The Cont is also attached directly on the site and performed obstructive attachment for 24 hours by using Dermapore.

### (2) Test Items

The test group to which the test preparation is administered for 21 days and the control group are subjected to (A) the measurement of weight, (B) the measurement of fed amounts, (C) pathologic test, (D) hematological test, aggregation time test, and biochemical test.

### (A) The measurement of weight

The weight of the animal are measured on the beginning day of the test, and then measured twice a week in morning (from 8:30 to 10:06 in morning) by using the precision balances (PB3001, Mettler-Toredo K. K.).

### (B) The measurement of fed amounts

The fed amounts are measured for all of cages once a week in morning (from 9:19 to 10:06 in morning) by using the precision balances (PB3001, Mettler-Toredo K. K.).

### (C) Pathologic test

Rats are sacrificed in bleeding on the day after the final administration date (16 hour starvation) by using sodium pentobarbital (30mg/kg, i.p., Dynabot Co., Ltd.) under anesthesia.
Subsequently, all the animals are subjected to macroscopic observation of body surfaces, openings, intracranial parts, cavitas thoracis, cavitas abdominalis and their contents. Also liver, kidney, adrenal gland, ovary and testis are removed, and measured by using the auto balance. Among them, kidney, ovary and testis are weighed as a pair.
In pathological test, dissected liver, kidney, adrenal gland, ovary and testis from all the animals are embedded in paraffin according to the conventional method to prepare sections, and stained by using hemato-eosin; and then subjected to the microscopic test.

### (D) Hematological test, Aggregation time test, and biochemical test

When the bleeding at (C) described above, blood sample are obtained for the hematological test and the biochemical test. Under the anesthesia of pentobarbital and laparotomy, needle for blood collection with a silicone tube is inserted into abdominal aorta, collected the blood form the natural blood shedding. The following item shown in Tables 7 and 8 are tested.

**(Table 7)**

| Examinations | Abbr. | Examination method |
|---|---|---|
| Number of red blood cells | RBC | DC detection method |
| Amount of | Hgb | SLS hemoglobin method |
| Hematocrit value | Hct | Erythrocyte puls wave high value detection method |
| Mean corpuscular volume | MCV | Calculation method |
| Mean corpuscular hemoglobin | MCH | Calculation method |
| Mean corpuscular hemoglobin concentration | MCHC | Calculation method |
| Platelet | Plt | DC detection method |
| Number of White blood cell | WBC | DC detection method |
| Reticulocyte | Reti | Brecher method |
| Ratio of leukocyte | | Light - Giemsa staining |
| Prothrombin time | PT | Viscosity change sensing system |
| Activated partial thromboplastin formation time | APTT | Viscosity change sensing system |

**(Table 8)**

| Examinations | Abbreviation | Examination method |
|---|---|---|
| Total bilirubin | T-Bil | Enzyme method |
| GOT | | JSCC matched method |
| GPT | | JSCC matched method |
| γ - GTP | | IFCC matched method |
| Cholinesterase | Chef | BTC·DTNB method |
| ALP | | p-nitrophenyl phosphate substrate method |
| Total protein | TP | Biuret method |
| A/G ratio | | Calculation method |
| Albumin | Alb | BCG method |
| Globulin | Glb | Calculation method |
| Total cholesterol | T-chow | CHO/DAOS method |
| Triglyceride | TG | GPO/POD method |
| Blood sugar | Glu | Hexokinase-G-6-PDF method |
| Urea nitrogen | BUN | Urease-GLDH method |
| Creatinine | Crea | Enzyme method |
| Sodium | Na | Electrode method |
| Potassium | K | Electrode method |
| Chlorine | Cl | Electrode method |
| Calcium | Ca | O-CPC method |
| Inorganic Phosphate | IP | Enzyme method |

### (4) Results

### (A) Weight measurement

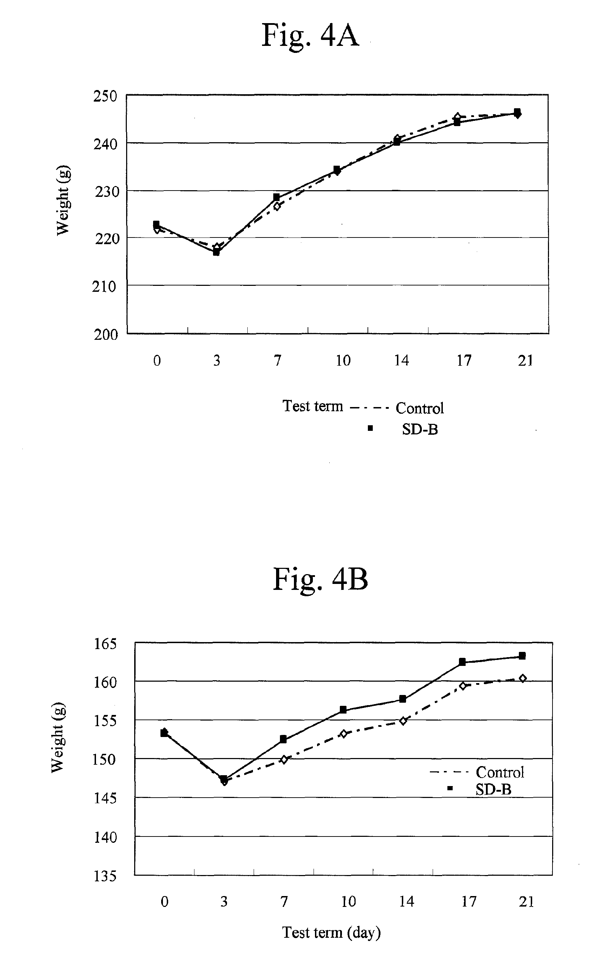

Weight change of each animal in the control groups and test groups are shown in Figs. 4A and 4B. Any animals do not loose their weight without relationship to sex. Alternatively, as shown in the table 9, any changes of the general state are observed through the test term, 21 days.

**(Table 9)**

| Sex | Groups | Number of animals | Symptom | General state (days after administration) | | | |
|---|---|---|---|---|---|---|---|
| | | | | 0 | 1-6 | 7-13 | 14-21 |
| Male | Control | 5 | Not abnormal | 5 | 5 | 5 | 5 |
| | Test | 5 | Not abnormal | 5 | 5 | 5 | 5 |
| Female | Control | 5 | Not abnormal | 5 | 5 | 5 | 5 |
| | Test | 5 | Not abnormal | 5 | 5 | 5 | 5 |

| | | | | | | | |
|---|---|---|---|---|---|---|---|
| * Numbers in the table show the animal number with no abnormalities. | | | | | | | |

### (B) Measurement of fed amounts

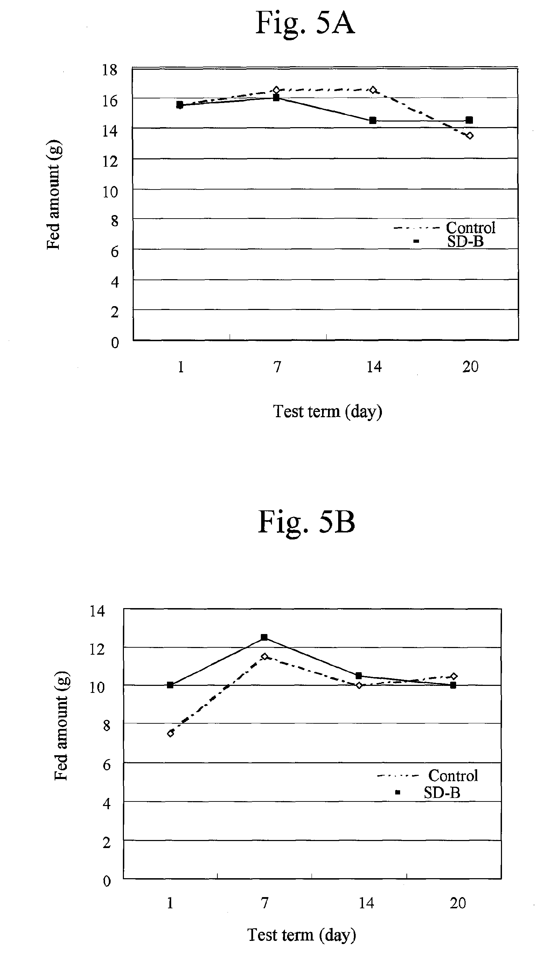

Results are shown in Fig. 5 in every male and female group. As shown in Figs. 5A and 5B, the fed amounts in test groups are not decreased compared with the control groups.

### (C) Pathologic test

Observation of body surfaces, openings, intracranial parts, cavitas thoracis, cavitas abdominalis and lymph nodes of the animals in control group and test group are shown in Table 10.

**(Table 10)**

| Observation Issues | Observation | Groups | | | |
|---|---|---|---|---|---|
| | | Male | | Female | |
| | | Control | Test | Control | Test |
| Body surface | Not abnormal | 5/5 | 5/5 | 5/5 | 5/5 |
| Intracranial parts | Not abnormal | 5/5 | 5/5 | 5/5 | 5/5 |
| Cavitas thoracis | Not abnormal | 5/5 | 5/5 | 5/5 | 5/5 |
| Cavitas abdominalis | Not abnormal | 5/5 | 5/5 | 5/5 | 5/5 |
| Lymph nodes | Not abnormal | 5/5 | 5/5 | 5/5 | 5/5 |

| | | | | | |
|---|---|---|---|---|---|
| * Numbers in the table show the animal number with no abnormalities. | | | | | |

As shown in Table 10, in macroscopy, any pathological changes are observed on the body surfaces, the openings, the intracranial parts, the cavitas thoracis, the cavitas abdominalis and the lymph nodes.

Next, Liver, kidney, adrenal gland, ovary and testis are removed from each animal in the control group and test group, and subjected to macroscopic observation. These organs are weighed by using an automatic balance to check weight changes. Results are shown in Tables 11 and 12.

**(Table 11)**

| Organs | Observation | | Groups | | | |
|---|---|---|---|---|---|---|
| | | | Male | | Female | |
| | | | Cont | Test prep. | Cont | Test prep. |
| Liver | No remarkable change | - | 3/5 | 4/5 | 5/5 | 5/5 |
| | Granulation nidus | + | 2/5 | 1/5 | 0/5 | 0/5 |
| Kidney | No remarkable change | - | 0/5 | 0/5 | 5/5 | 5/5 |
| | Acidophil corpscle in Proximal uriniferous tuble | + | 5/5 | 5/5 | 0/5 | 0/5 |
| | Basophil uriniferous tuble | ± | 5/2 | 0/5 | 0/5 | 0/5 |
| | | + | 1/5 | 0/5 | 0/5 | 0/5 |
| Adrenal Gland | No remarkable change | - | 5/5 | 5/5 | 5/5 | 5/5 |
| Testis | No remarkable change | - | 5/5 | 5/5 | - | - |
| Ovary | No remarkable change | - | - | - | 5/5 | 5/5 |

**(Table 12)**

| Organs | Groups | | | |
|---|---|---|---|---|
| | Male | | Female | |
| | Cont | Test preparation | Cont | Test preparation |
| Liver | 6.612±0.706 | 6.800±0.763 | 4,394±0.157 | 4.318±0.242 |
| | (100.0) | (102.8) | (100.0) | (98.3) |
| Kidney | 1.792±0.137 | 1.736±0.763 | 1.246±0.017 | 1.218±0.025 |
| | (100.0) | (96.9) | (100.0) | (97.7) |
| Adrenal | 38.6±1.9 | 39.2 ±2.4 | 50.8±0.4 | 51.6±2.1 |
| Gland | (100.0) | (101.5) | (100.0) | (101.6) |
| Testis | 2.674±0.079 | 2.690±0.120 | - | - |
| | (100.0) | (100.6) | - | - |
| Ovary | - | - | 54.2±3.4 | 56.6±2.9 |
| | - | - | (100.0) | (104.4) |

As shown in Table 11, even in either male or female group, any groups do not show pathosis in any organs compared with each control groups. Alternatively, as shown in Table 12, no significant change is observed in liver, kidney, adrenal gland, ovary and testis removed.

Results of the hematological tent and biochemical test of the control group and the test group are shown in Tables 13 to 16, classifying male and female group.

**(Table 13)**

| Female | Dose | |
|---|---|---|
| | Cont | SD-B |
| Number of Animals | (5) | (5) |
| RBC (×10⁴/µL) | 934.6 ± 36.2 | 934.8 ± 52.9 |
| Hgb (g / dL) | 16.66 ± 0.83 | 16.42 ± 0.64 |
| Hct (%) | 48.90 ± 2.25 | 48.82 ± 2.64 |
| MCV (fL) | 52.30 ± 0.45 | 52.22 ± 0.34 |
| MCH (pg) | 17.82 ± 0.22 | 17.60 ± 0.33 |
| MCHC (%) | 34.06 ± 0.40 | 33.64 ± 0.61 |
| PLT (×10⁴/µL) | 94.62 ± 5.35 | 93.16 ± 15.94 |
| WBC (×10²/µL) | 56.6 ± 14.2 | 49.2 ± 11.0 |
| STAB (%) | 0.0 ± 0.0 | 0.0 ± 0.0 |
| SEG (%) | 27.2 ± 5.8 | 25.6 ± 7.2 |
| LYMPH (%) | 72.8 ± 5.8 | 74.4 ± 7.2 |
| MONO (%) | 0.0 ± 0.0 | 0.0 ± 0.0 |
| BASO (%) | 0.0 ± 0.0 | 0.0 ± 0.0 |
| EOSINO (%) | 0.0 ± 0. 0 | 0.0 ± 0.0 |
| Reti (‰) | 18.0 ± 2.4 | 21.0 ± 3.2 |
| PT (sec) | 16.78 ± 1.16 | 16.50 ± 0.92 |
| APTT (sec) | 13.74 ± 1.21 | 13.68 ± 0.97 |
| Mean ± S.D. | | |

**(Table 14)**

| Female | Dose | |
|---|---|---|
| | Control | SD-B |
| Number of Animals | (5) | (5) |
| RBC (×10⁴/µL) | 838.8 ± 17.8 | 868.0 ± 30.9 |
| Hgb (g/dL) | 15.46 ± 0.22 | 15.98 ± 0.61 |
| Hct (%) | 44.64 ± 1.00 | 46.22 ± 1.98 |
| MCV (fL) | 53.20 ± 0.20 | 53.24 ± 0.50 |
| MCH (pg) | 18.42 ± 0.18 | 18.40 ± 0.19 |
| MCHC (%) | 34.64 ± 0.38 | 34.60 ± 0.50 |
| PLT (×10⁴/µL) | 84.34 ± 4.69 | 85.92 ± 3.66 |
| WBC (×10²/µL) | 42.2 ± 5.4 | 39.2 ± 8.7 |
| STAB (%) | 0.0 ± 0.0 | 0.0 ± 0.0 |
| SEG (%) | 25.6 ± 3.0 | 22.8 ± 2.6 |
| LYMPH (%) | 74.4 ± 3.0 | 77.2 ± 2.6 |
| MONO (%) | 0.0 ± 0.0 | 0.0 ± 0.0 |
| BASO (%) | 0.0 ± 0.0 | 0.0 ± 0.0 |
| EOSINO (%) | 0.0 ± 0.0 | 0.0 ± 0.0 |
| Reti (‰) | 19.6 ± 2.2 | 17.2 ± 1.9 |
| PT (sec) | 16.10 ± 0.89 | 15.18 ± 0.29 |
| APTT (sec) | 11.82 ± 0.43 | 12.32 ± 0.73 |
| Mean ± S.D. | | |

As shown in Tables 13 and 14, there is no significant difference on the result of the hematological test between the control group and the sample administered one.

**(Table 15)**

| Female | Dose | |
|---|---|---|
| | Control | SD-B |
| Number of animals | (5) | (5) |
| T-Bil (mg/dl) | 0.058 ± 0.013 | 0.052 ± 0.016 |
| GOT (IU/L) | 114.4 ± 7.3 | 108.4 ± 6.6 |
| GPT (IU/L) | 77.2 ± 8.1 | 70.6 ± 5.2 |
| γ-GTP (IU/L) | 0.2 ± 0.4 | 0.0 ± 0.0 |
| ChE (IU/L) | 94.8 ± 6.3 | 86.8 ± 5.2 |
| ALP (IU/L) | 435.0 ± 36.9 | 391.4 ± 52.8 |
| TP (g/dL) | 5.98 ± 0.15 | 5.84 ± 0.22 |
| A/G | 1.452 ± 0.061 | 1.498 ± 0.051 |
| Alb (g/dL) | 3.54 ± 0.05 | 3.50 ± 0.12 |
| Glb (g/dL) | 2.44 ± 0.11 | 2.34 ± 0.11 |
| T-cho (mg/dL) | 61.0 ± 4.4 | 60.2 ± 5.2 |
| TG (mg/dL) | 49.6 ± 13.6 | 68.0 ± 16.8 |
| Glu (mg/dL) | 154.4 ± 25.1 | 154.8 ± 21.7 |
| BUN (mg/dL) | 26.96 ± 1.42 | 26.06 ± 1.16 |
| Crea (mg/dL) | 0.34 ± 0.05 | 0.30 ± 0.00 |
| Na (mmol/L) | 141.8 ± 0.8 | 142.2 ± 1.3 |
| K (mmol/L) | 4.58 ± 0.54 | 4.94 ± 0.15 |
| Cl (mmol/L) | 106.2 ± 1.3 | 107.2 ± 0.8 |
| Ca (mg/dL) | 10.70 ± 0.46 | 10.66 ± 0.13 |
| IP (mg/dL) | 7.04 ± 0.53 | 7.44 ± 0.55 |
| Mean ± S.D. | | |

**(Table 16)**

| Female | Dose | |
|---|---|---|
| | Control | SD-B |
| Number of Animals | (5) | (5) |
| T-Bil (mg/dl) | 0.072 ± 0.019 | 0.064 ± 0.017 |
| GOT (IU/L) | 92.0 ± 6.2 | 97.4 ± 2.7 |
| GPT (IU/L) | 63.4 ± 6.7 | 59.6 ± 4.2 |
| γ-GTP (IU/L) | 0.6 ± 0.5 | 0.4 ± 0.5 |
| ChE (IU/L) | 817.8 ± 78.8 | 821.4 ± 105.7 |
| ALP (IU/L) | 351.0 ± 35.7 | 313.8 ± 32.0 |
| TP(g/dL) | 5.50 ± 0.14 | 5.54 ± 0.11 |
| A/G | 1.570 ± 0.014 | 1.504 ± 0.120 |
| Alb (g/dL) | 3.36 ± 0.09 | 3.34 ± 0.09 |
| Glb (g/dL) | 2.14 ± 0.05 | 2.20 ± 0.14 |
| T-cho (mg/dL) | 68.6 ± 4.6 | 63.6 ± 8.2 |
| TG (mg/dL) | 31.4 ± 5.4 | 28.0 ± 9.6 |
| Glu (mg/dL) | 146.2 ± 13.1 | 135.0 ± 20.5 |
| BUN (mg/dL) | 29.16 ± 2.17 | 28.04 ± 3.15 |
| Crea (mg/dL) | 0.30 ± 0.00 | 0.30 ± 0.00 |
| Na (mmol/L) | 142.6 ± 0.5 | 141.8 ± 1.1 |
| K (mmol/L) | 4.20 ± 0.23 | 4.32 ± 0.25 |
| Cl (mmol/L) | 108.6 ± 0.9 | 108.2 ± 1.1 |
| Ca (mg/dL) | 10.28 ± 0.15 | 10.50 ± 0.25 |
| IP (mg/dL) | 7.28 ± 0.56 | 7.54 ± 0.62 |
| Mean ± S.D. | | |

In either of the hematological test or biochemical test, there is no significant difference between the test groups and the control groups.
Accordingly, since the sleep inducer of the present invention has quite low level of irritation properties, and no percutaneous toxicity, it is demonstrated that the sleep inducer of the present invention has high safety.

### INDUSTRIAL APPLICABILITY

As described before, the sleep inducer of the present invention is useful in the field of medicaments for treating disease such as insomnia, because sleep elongation effect when the duration of loss of righting reflex is employed as the index.

## Claims

1. A sleep inducing composition comprising geranyl acetate type Eucalyptus oil as an active ingredient.

2. A sleep inducing composition as claimed in claim 1, wherein a water absorption resin is covered by said geranyl acetate type Eucalyptus oil to prepare an essential oil covered particle, and then the particle is further covered by a carbon fine powder, which functions as an essential oil desorbing regulator, to prepare a carbon coated particle that is covered.

3. A sleep inducer as claimed in claim 2, comprising geranyl acetate type Eucalyptus oil, an essential oil adsorbent, a free-water remover, an essential oil adsorbing and desorbing regulator, an exothermic agent, a heat transfer inhibitor, an absorption promoter, a base material for sheet forming, and a sheet for contact bonding.

4. A sleep inducer as claimed in claim 3, wherein said essential oil adsorbent is a polyvinyl alcohol type water absorption resin of which saponification value is from 98.0 to 98.5.

5. A sleep inducer as claimed in claim 3, wherein said free-water remover is an acrylic type water-absorptive resin.

6. A sleep inducer as claimed in claim 5, wherein said acrylic type water-absorptive resin has a high-molecular-weight which is capable of absorbing 400 to 800 times its volume of water based on the volume of the water-absorptive acrylic resin.

7. A sleep inducer as claimed in claim 3, wherein said an essential oil adsorbing and desorbing regulator is a porous carbon material having surface area from 200 to 800 m².

8. A sleep inducer as claimed in claim 3, wherein said exothermic agent is a zeolite having a pore size from 0.1 to 0.8 nm.

9. A sleep inducer as claimed in claim 3, wherein said heat transfer inhibitor is a polysaccharide compound.

10. A sleep inducer as claimed in claim 3, wherein said absorption promoter is mono terpene compound.

11. A sleep inducer as claimed in claim 10, wherein said monoterpene compound is 1-menthol or limonene.

12. A sleep inducer as claimed in claim 3, wherein said base material for sheet forming is a thermoplastic resin having about 88.0 as the saponification value.

13. A plaster type of a sleep inducer comprising geranyl acetate type Eucalyptus oil, an essential oil adsorbing material, an essential oil adsorbing and desorbing agent, and an absorption promoter.

14. A plaster type of a sleep inducer as claimed in claim 13, wherein said composition comprising geranyl acetate type Eucalyptus oil, an essential oil adsorbing material is a finely divided activated carbon particles.

15. A plaster type of a sleep inducer as claimed in claim 13, wherein said absorption promoter is 1-menthol or limonene.

16. A plaster type of a sleep inducer as claimed in claim 13, wherein said base material is an oleaginous base selected from a group consisting of fatty acid ester such as lard, beef tallow, fatty oil, paraffin having branched chain, solid paraffin, white soft paraffin, hexanoic acid, enanthic acid, caprylic acid, pelargonic acid, undecylic acid, lauric acid, tridecylic acid, myristic acid, pentadecylic acid, palmitic acid, heptadecylic acid, stearic acid, oleic acid, myristyl palmitate, stearyl stearate, myristic myristate, ceryl lignocerate: lanolin, lately describing waxes; glyceryl laurate, glyceryl monomyristate, glyceryl monooleate, glyceryl monostearate, glyceryl dilaurate, glyceryl dimyristate, glyceryl distearate, glyceryl trilaurate, glyceryl trimyristate, glyceryl tristearate; soybean oil, camellia oil, rape seed oil, peanut oil, sesame oil, safflower oil, mink oil, egg yolk oil, squarane, fish oil, whale oil, liver oil, carnauba wax, yellow beeswax, and white beewax.

17. A plaster type of a sleep inducer as claimed in claim 13, wherein said base material is a water-soluble base selected from the group consisting of carboxyvinylpolymer, hydroxypropylcellulose, macrogol, and methylcellulose.

18. A plaster type of a sleep inducer as claimed in claim 17, wherein diisopropylethanoamine and diisopropyladipate are used as neutralization agent, when said carboxyvinylpolymer is used as said water-soluble base.

19. A method for producing a sleep inducer comprising steps of
weighing predetermined weight of geranyl acetate type Eucalyptus oil,
mixing the oil with an essential oil adsorbent to allow a coating of the essential oil on the essential oil adsorbent,
adding a porous carbon material, which adsorbs and desorbs an essential oil, to an essential oil-coated adsorbent to allow a carbon coated particle being coated by the porous carbon material,
mixing homogeneously a predetermined amount of the carbon coated particle, an exothermic agent, a heat transfer inhibitor, and a base material for sheet forming, tot prepare a layer having even thickness, and then heated them to form a composition for sleep inducing agent,
cutting said composition for sleep inducing agent, which is percutaneous agent, in a predetermined size of a piece to sandwich it by using two sheet type materials, and
thermal bonding of four sides of the sheet type materials.

20. A method for producing a plaster type sleep inducer comprising the steps of:
weighing predetermined weight of geranyl acetate type Eucalyptus oil,
mixing the oil with an essential oil adsorbent to allow a coating of the essential oil on the essential oil adsorbent,
adding a porous carbon material, which adsorbs and desorbs an essential oil, to an essential oil-coated adsorbent to allow a carbon coated particle being coated by the porous carbon material,
mixing homogeneously a predetermined amount of the carbon coated particle, an absorption promoter, and either one of an oleaginous base or water-soluble base to form a mixture thereof, and
spreading the mixture on a sheet made of paper or plastic.
